# EUROPEAN PATENT APPLICATION

(11) **EP 2 921 231 A1**
(43) Date of publication of application: **23.09.2015**
(21) Application number: 15159367.0
(22) Date of filing: 17.03.2015
(51) Int. Cl.: B01L 3/00

(54) **TARGET SUBSTANCE PURIFICATION DEVICE, NUCLEIC ACID PURIFICATION DEVICE, TARGET SUBSTANCE GENERATING METHOD, AND NUCLEIC ACID AMPLIFICATION METHOD**

(30) Priority: 19.03.2014 JP 2014057146
(71) Applicant: Seiko Epson Corporation, Tokyo 163 (JP)
(72) Inventor: Murayama, Toshiro, Nagano, 392-8502 (JP); Takagi, Fumio, Nagano, 392-8502 (JP); Saito, Yuji, Nagano, 392-8502 (JP)
(74) Representative: Hoffmann Eitle

(57) **Abstract**

A target substance purification device includes: a capillary that has a longitudinal direction and includes a plug of oil, a plug of an elution liquid that undergoes phase separation from oil, and elutes the target substance from a magnetic body detachably retaining the target substance, and a plug of oil in this order inside the capillary; a magnetic force applying mechanism that applies a magnetic force to the capillary to retain the magnetic body, and control the movement of the magnetic body along the longitudinal direction of the capillary; and a liquid sending mechanism that moves the elution liquid in the longitudinal direction of the capillary while the magnetic force applying mechanism is restricting the movement of the magnetic body along the longitudinal direction of the capillary.

## Description

### BACKGROUND

### 1. Technical Field

The present invention relates to a target substance purification device, a nucleic acid purification device, a target substance generating method, and a nucleic acid amplification method.

### 2. Related Art

Boom et al. reports a method for conveniently extracting nucleic acids from biological materials with the use of a nucleic acid-binding solid phase support such as silica particles, and a chaotropic agent (J. Clin. Microbiol., vol. 28 No.3, p. 495-503 (1990)). The method of Boom et al., and all the other extraction methods that involve adsorbing a nucleic acid on a support with a nucleic acid-binding solid phase support (such as silica) and a chaotropic agent basically include the steps of (1) adsorbing a nucleic acid on a nucleic acid-binding solid phase support in the presence of a chaotropic agent (adsorbing step), (2) washing the support and the adsorbed nucleic acid with a washing liquid to remove the non-specifically bound foreign substances and the chaotropic agent (washing step), and (3) eluting the nucleic acid from the support with water or a low-salt buffer (eluting step), as described, for example, in JP-A-11-146783, and JP-A-2009-207459.

A problem of such methods, however, is that each support particle is a very fine magnetic particle, and its hydrophilic surface may cause some of the particles to remain in the aqueous solution elution liquid when the externally applied magnetic procedures are insufficient. The presence of such magnetic particle residues in the elution liquid may interfere with the subsequent detection of a reaction by blocking the excitation light applied for detecting an amplification reaction.

### SUMMARY

An advantage of some aspects of the invention is to provide a target substance purification device, a nucleic acid purification device, a target substance generating method, and a nucleic acid amplification method with which a magnetic body can be efficiently removed.

An aspect of the invention is directed to a target substance purification device that includes: a capillary that has a longitudinal direction and includes a plug of oil, a plug of an elution liquid that undergoes phase separation from oil, and elutes the target substance from a magnetic body detachably retaining the target substance, and a plug of oil in this order inside the capillary; a magnetic force applying mechanism that applies a magnetic force to the capillary to retain the magnetic body, and control the movement of the magnetic body along the longitudinal direction of the capillary; and a liquid sending mechanism that moves the elution liquid in the longitudinal direction of the capillary while the magnetic force applying mechanism is restricting the movement of the magnetic body along the longitudinal direction of the capillary. The purification device may include a mount section that is adapted to mount the capillary. The magnetic force applying mechanism may include a permanent magnet. The liquid sending mechanism may include a pressure applying member. The pressure applying member may be a plunger.

Another aspect of the invention is directed to a nucleic acid purification device that includes a capillary that has a longitudinal direction and includes an oil plug of oil, a washing liquid plug of a washing liquid, an oil plug of oil, an elution liquid plug of an elution liquid that undergoes phase separation from oil, and elutes a nucleic acid from a magnetic body detachably retaining the nucleic acid, and an oil plug of oil in this order inside the capillary; a magnetic force applying mechanism that applies a magnetic force to the capillary to retain the magnetic body, and control the movement of the magnetic body along the longitudinal direction of the capillary; and a liquid sending mechanism that moves the elution liquid plug along the longitudinal direction of the capillary while the magnetic force applying mechanism is restricting the movement of the magnetic body along the longitudinal direction of the capillary. The magnetic force applying mechanism may move the magnetic body within a plane that crosses the longitudinal direction of the capillary. The nucleic acid purification device may include a nucleic acid amplification vessel that is in communication with inside of the capillary. The nucleic acid amplification vessel may contain a freeze dried nucleic acid amplification reagent.

Still another aspect of the invention is directed to a method for purifying a target substance, the method including: introducing a magnetic body retaining the target substance into a capillary that has a longitudinal direction and includes a plug of oil, an elution liquid plug of an elution liquid that undergoes phase separation from oil, and elutes the target substance from a magnetic body detachably retaining the target substance, and a plug of oil in this order inside the capillary; externally applying a magnetic force to the capillary to move the magnetic body into the elution liquid plug and retain the magnetic body inside the elution liquid plug; eluting the target substance from the magnetic body in the liquid; and moving the liquid with the eluted target substance out of the capillary in the longitudinal direction of the capillary while restricting the movement of the magnetic body along the longitudinal direction of the capillary. The purification method may include moving the magnetic body within a plane that crosses the longitudinal direction of the capillary. The liquid with the eluted target substance may be moved in the longitudinal direction of the capillary by increasing the pressure inside the capillary. The pressure inside the capillary may be increased with a plunger.

Yet another aspect of the invention is directed to a method for amplifying a nucleic acid obtained as the target substance by using any of the foregoing purification methods.

The aspects of the invention have thus enabled providing a target substance purification device, a nucleic acid purification device, a target substance generating method, and a nucleic acid amplification method whereby a magnetic body can be efficiency removed.

### BRIEF DESCRIPTION OF THE DRAWINGS

The invention will be described with reference to the accompanying drawings, wherein like numbers reference like elements.
FIGS. 1A and 1B are schematic diagrams of a nucleic acid purification device according to an embodiment of the invention.
FIG. 2 is a magnified view near a plug inside a capillary according to the embodiment of the invention.
FIG. 3 is a schematic diagram of the nucleic acid purification device with a reaction vessel and a substance extraction unit according to the embodiment of the invention.
FIGS. 4A and 4B are schematic diagrams representing a use of the nucleic acid purification device according to the embodiment of the invention.
FIGS. 5A to 5F are schematic diagrams representing a specific procedure of using the nucleic acid purification device according to the embodiment of the invention.
FIG. 6 is a schematic diagram of the nucleic acid purification device according to the embodiment of the invention.
FIGS. 7A to 7C are schematic diagrams showing how a solution dissolving a specimen is introduced into the nucleic acid purification device in the nucleic acid purification method according to the embodiment of the invention.
FIGS. 8A to 8D are schematic diagrams showing how magnetic particles are moved into the elution liquid while being swung according to the nucleic acid purification method according to the embodiment of the invention.
FIGS. 9A to 9C are schematic diagrams showing how the elution liquid is pushed out of the capillary according to the nucleic acid purification method according to the embodiment of the invention.

### DESCRIPTION OF EXEMPLARY EMBODIMENTS

The objects, features, advantages, and ideas of the invention will be clearly understood by a skilled person from the descriptions of the invention, and it would be easy for a skilled person to reproduce the invention from the following descriptions. The embodiments and concrete examples of implementation discussed in the following detailed explanation serve solely to illustrate or describe the preferred embodiments of the invention, and do not limit the invention in any ways. It will be understood that various modifications and alterations may be made to the invention by a skilled person from the following descriptions, provided such modifications and alterations do not exceed the intent and the scope of the invention disclosed below.

### Target Substance Purification device

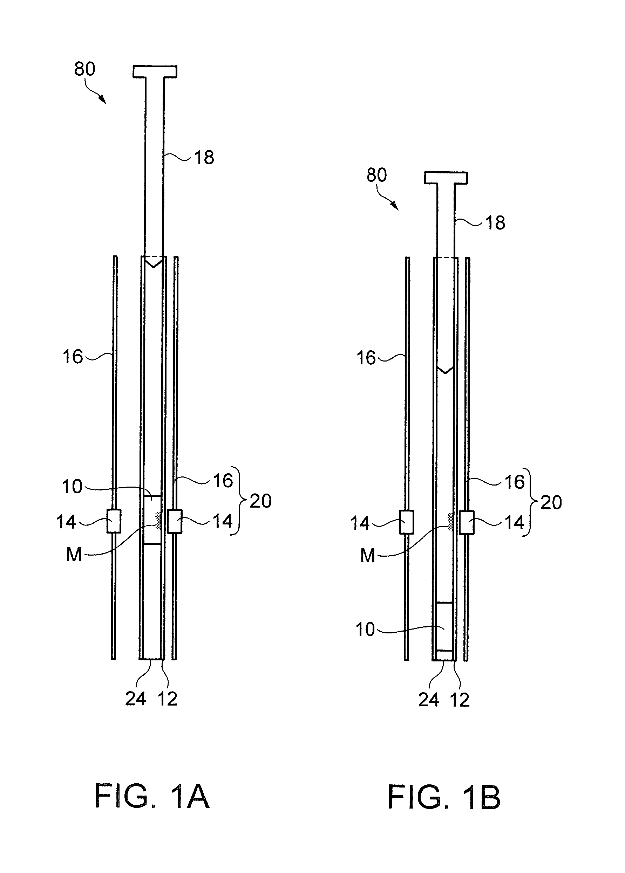

As illustrated on FIG. 1A, an embodiment of a target substance purification device 80 according to the invention includes a capillary 12 having a liquid plug 10, a magnetic body M detachably retaining a target substance inside the capillary 12, a magnetic force applying mechanism 20 that applies a magnetic force to the capillary 12 to retain the magnetic body M, and control the movement of the magnetic body M along the longitudinal direction of the capillary 12, and a liquid sending mechanism 18 that moves the liquid along the longitudinal direction of the capillary 12 while the magnetic force applying mechanism 20 is restricting the movement of the magnetic body M.

The liquid plug 10 is preferably an aqueous solution, more preferably a salt solution, particularly a buffer. The capillary 12 may have more than one plug. In this case, it is preferable to provide a wax plug or an oil plug between the liquid plugs so that the liquid plugs can independently exist.

The capillary 12 is hollow inside, and has a tubing portion (also called "tube portion") that allows a liquid to longitudinally travel inside the hollow space. The tube portion has a longitudinal direction, but may be bent. The size and the shape of the hollow space inside the tube portion are not particularly limited, as long as the liquid can maintain the form of a plug inside the tube portion. The size of the hollow space inside the tube portion, and the shape of the cross section perpendicular to the longitudinal direction may vary along the longitudinal direction of the tube portion. Whether the liquid can maintain a plug shape inside the tube portion depends on conditions such as the material of the tube portion, and the type of the liquid, and as such the shape of the cross section perpendicular to the longitudinal direction of the tube portion is appropriately designed to allow the liquid to maintain a plug shape inside the tube portion. The outer cross sectional shape of the tube portion perpendicular to the longitudinal direction is not limited either. The thickness of the tube portion is not particularly limited. When the cross section of the hollow space inside the tube portion perpendicular to the longitudinal direction is circular in shape, the tube portion may have an inner diameter (the diameter of the circle of the cross section of the inner hollow perpendicular to the longitudinal direction) of, for example, 0.5 mm to 3 mm. Such an inner diameter of the tube portion is preferable in terms of ease of forming a liquid plug over a wide range of tube portion materials and liquid types. The material of the tube portion is not particularly limited, and may be, for example, glass, polymer, or metal. The materials selected for the tube portion are preferably materials that are transparent to visible light, such as glass and polymer, because such materials provide visual access to inside of the tube portion (the hollow space) from outside. It is also preferable to use a magnetically transparent material or a non-magnetic material for the tube portion because such materials make it easier to externally apply a magnetic force to the tube portion such as when passing the magnetic body M through the tube portion.

The target substance is not limited, and may be a macromolecule such as a nucleic acid (e.g., DNA, RNA), and a protein, or a low-molecular substance such as a compound. The shape of the magnetic body M is not particularly limited, and is preferably particulate. In order to detachably retain the target substance, the magnetic body M either detachably binds to the target substance, or has a target substance-binding substance that detachably binds to the target substance. For example, when the target substance is a nucleic acid, the magnetic body M may have nucleic acid binding molecules such as silica, glass, and diatomaceous earth. The magnetic body M may have an antibody binding molecule such as protein A when the target substance is an antibody.

The purification device 80 of the embodiment of the invention includes a magnetic force applying mechanism 20. The magnetic force applying mechanism 20, provided as a mechanism for externally applying a magnetic force to the capillary 12 to retain the magnetic body M and control the movement of the magnetic body M inside the capillary 12 may easily be designed by a skilled person. For example, the magnetic force applying mechanism 20 has a magnetic force applying body 14, such as a permanent magnet, that is supported by an applying body supporting section 16, and can retain the magnetic body M with the magnetic force of the magnetic force applying body 14. The magnetic force applying mechanism 20 controls the longitudinal movement of the magnetic body M inside the capillary 12, and can stop the magnetic body M as desired. This is made possible, for example, with a mechanism by which the magnetic force applying body 14 can be moved, and stopped at the desired position. Preferably, the magnetic force applying mechanism 20 includes a moving mechanism that moves the magnetic body M within a plane that crosses the longitudinal direction of the capillary 12, particularly a plane orthogonal to the longitudinal direction of the capillary 12. Specifically, it is preferable that the magnetic force applying mechanism 20 be adapted to be capable of swinging the magnetic body M. For example, when a pair of magnetic force applying bodies 14 is provided as the moving mechanism, the magnetic body M is attracted by whichever of the magnetic force applying bodies 14 is closer to the magnetic body M, and the magnetic force applying body 14 that is more proximate than the other to the capillary 12 attracts the magnetic body M. As the magnetic force applying body 14 is moved away from the capillary 12, the magnetic body M is attracted by the other magnetic force applying body 14 approaching the capillary 12 from the opposite side. In this way, the magnetic body M can be moved sideways. The magnetic body M can thus be moved back and forth sideways by swinging the pair of magnetic force applying bodies 14 sideways. This makes it easier for the magnetic body M to contact the liquid as it moves in the liquid plug 10, and improves the washing and eluting effects.

The liquid sending mechanism 18 of the purification device 80 is provided to move the liquid plug 10 along the longitudinal direction of the capillary 12, and eject the liquid through a capillary end portion 24. For example, the liquid sending mechanism 18 may be a pressure applying member that applies pressure to the liquid inside the capillary 12, and moves the liquid under the applied pressure, or a suction member that moves the liquid by evacuating the capillary 12 and creating a negative pressure therein. The pressure applying member may be, for example, a piston or a plunger as shown in FIGS. 1A and 1B. The suction member may be, for example, a vacuum pump. Preferably, the diameter of the piston or the plunger is the same as the inner diameter of the capillary 12, and the piston or the plunger is directly fitted to the capillary 12. In this case, as illustrated on FIG. 1B, the liquid plug can be moved along the longitudinal direction of the capillary 12 by pushing the piston or the plunger into the capillary 12. In the case of a vacuum pump, for example, the capillary 12 may be fitted to the opening of a side arm flask through a rubber stopper, and a vacuum may be created inside the flask with a vacuum pump such as an aspirator connected to the arm. The liquid plug also can be moved along the longitudinal direction of the capillary 12 in this manner. Alternatively, a detachable sealing member such as a cap may be provided at the both ends of the capillary 12, and may be opened and closed to move the liquid with the eluted target substance along the longitudinal direction of the capillary 12 under the force of gravity, and discharge and collect the liquid from the capillary 12 held vertical to the ground. It is also possible to use the capillary 12 provided at one end with a high volatility liquid plug that does not instantaneously mix with oil. Heating the plug after sealing this end of the capillary 12 vaporizes the high volatility liquid, and moves the liquid with the eluted target substance toward the opposite end from the high volatility liquid plug along the longitudinal direction of the capillary 12.

### Target Substance Purification Method

The purification device 80 of the foregoing configuration may be used to purify a target substance. For example, a target substance of interest for purification is obtained from a sample such as cells and viruses containing the target substance, for example, by lysing or extracting the sample with a lysing solution or an extractant. The magnetic body M that can detachably retain the target substance is then added, and bound to the target substance. The magnetic body M detachably retaining the target substance may be washed with a buffer or the like with a centrifuge tube or the like. The magnetic body M detachably retaining the target substance is then introduced into the capillary 12 having the liquid plug 10. The method for introducing the magnetic body M is not particularly limited, and the magnetic body M may be introduced into the capillary 12 through the end portion 24 after being suspended in the same liquid used for the liquid plug 10 so that the liquids coalesce inside the capillary 12.

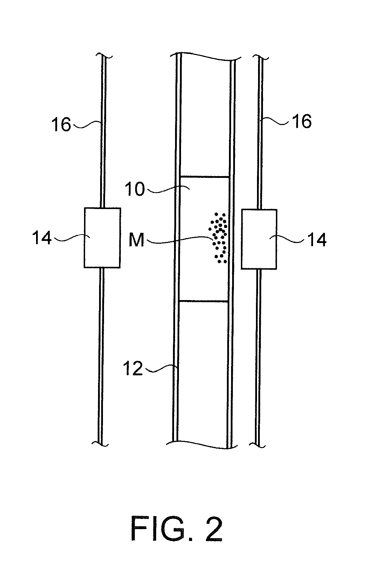

Simultaneously, a magnetic force is applied from outside of the capillary 12 to retain the magnetic body M inside the liquid. For example, as illustrated in FIG. 2, the magnetic body M may be retained inside the liquid by installing the magnetic force applying body 14 in proximity outside (right side) of the liquid plug 10. The magnetic body M may be moved as desired inside the capillary 12, or may be retained at the specific position along the longitudinal direction of the capillary 12 by moving the magnetic force applying body 14 along the longitudinal direction of the capillary 12, or by stopping the magnetic force applying body 14 as desired with the magnetic force applying mechanism 20.

With the magnetic body M retained inside the liquid, the target substance is eluted from the magnetic body M in the liquid. The elution method is not particularly limited, and may be appropriately decided according to the manner in which the target substance binds to the target substance-binding substance of the magnetic body M. For example, in the case of a nucleic acid and silica, the liquid may be heated by heating the capillary. In the case of an antibody and an antigen, the antibody and the antigen may be separated from each other by making the pH acidic by addition of an acid to the liquid. Here, the liquid effect on the magnetic body M can be improved by swinging the magnetic body M on a plane that crosses the longitudinal direction of the capillary 12, particularly a plane orthogonal to the longitudinal direction of the capillary 12, as described above.

Thereafter, as illustrated on FIG. 1B, the liquid with the eluted target substance is moved along the longitudinal direction of the capillary 12 , and ejected through the end 24 of the capillary 12 while stopping the longitudinal movement of the magnetic body M along the capillary 12. The liquid can then be collected to obtain the target substance that has eluted from the magnetic body.

More than one liquid plug may be provided, one being a washing liquid for washing the magnetic body M detachably retaining the target substance, and the other being an elution liquid for eluting the target substance from the magnetic body M. Specifically, in this case, the capillary 12 may include a first plug of oil, a second plug of a washing liquid that undergoes phase separation from the oil, and washes the magnetic body detachably retaining the target substance, a third plug of oil, a fourth plug of an elution liquid that undergoes phase separation from the oil, and elutes the target substance from the magnetic body M detachably retaining the target substance, and a fifth plug of oil, in this order inside the capillary 12. As with the case of a single plug, the magnetic body M detachably retaining the target substance is introduced into the washing liquid. The magnetic body M is then moved to the elution liquid with the magnetic force applying mechanism 20. The target substance is then eluted in the elution liquid, and is ejected and collected through the end of the capillary, as described above. Here, the washing effect can be further improved by providing more than one washing liquid plug.

### Example of Nucleic Acid Purification

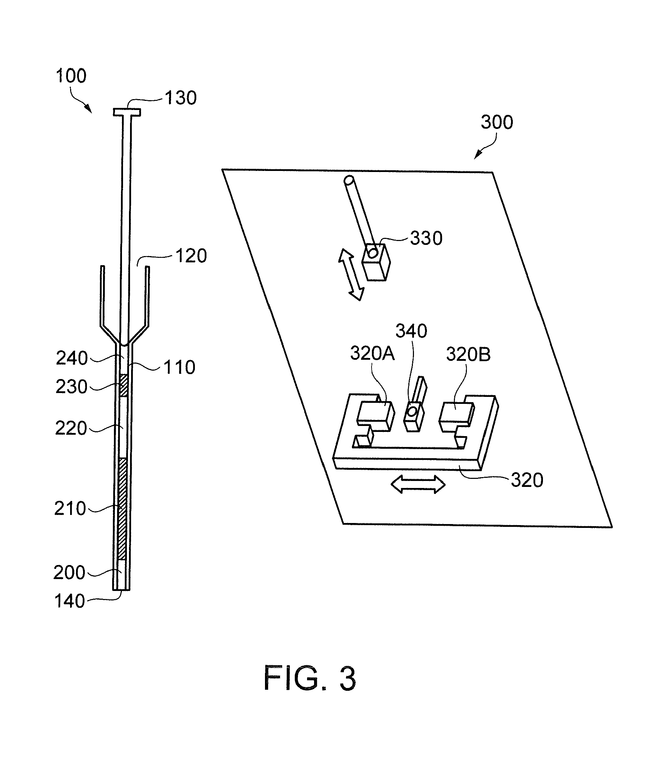

As illustrated in FIG. 3, the nucleic acid purification device of this Example includes a reaction vessel 100, and a substance extraction unit 300. The reaction vessel 100 includes the capillary 12 having a longitudinal direction, and a plunger 130 connected to the capillary 12 on the side of an opening 120 provided at one end of the capillary 12. The capillary 12 has an opening 140 at the end opposite the opening 120, and is charged with a first plug 200 of an oil, a second plug 210 of an washing liquid, a third plug 220 of an oil, a fourth plug 230 of an elution liquid, and a fifth plug 240 of an oil, in this order from the opening 140 toward the opening 120. The washing liquid and the elution liquid are aqueous solutions immiscible to the oil. The substance extraction unit 300 includes a mount section 310 for installing the reaction vessel 100, permanent magnets 320A and 320B for applying a magnetic force to the side surface of the capillary 12 of the reaction vessel 100, a liquid sending mechanism 330 that pushes the plunger 130 to send the liquid inside the reaction vessel 100, and a heating section 340 for heating a part of the capillary 12. In this Example, silicone oil was used as the oil. A 76 mass% guanidine hydrochloride aqueous solution was used as the washing liquid. Sterile water was used as the elution liquid.

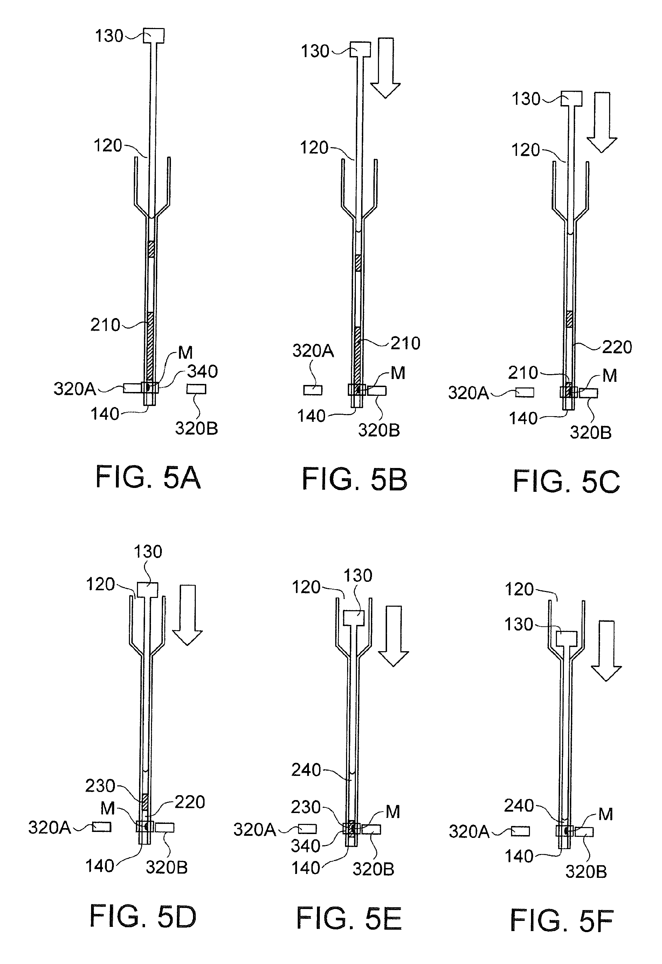

As used herein, "plug" refers to a specific liquid that compartmentalizes the inner space of the capillary 12. In FIGS. 5A to 5F, "plug" is the liquid retained in the form of a column. The oil undergoes phase separation upon being mixed with an aqueous solution, and accordingly the oil plug functions to prevent mixing of the water-soluble plugs situated on the both sides of the oil plug. Preferably, no bubbles or other liquids exist inside and between the plugs. However, bubbles or other liquids may be present as long as the magnetic particles M (described later) can pass through the plug.

The following describes how the nucleic acid purification device is used to extract a nucleic acid from human blood.

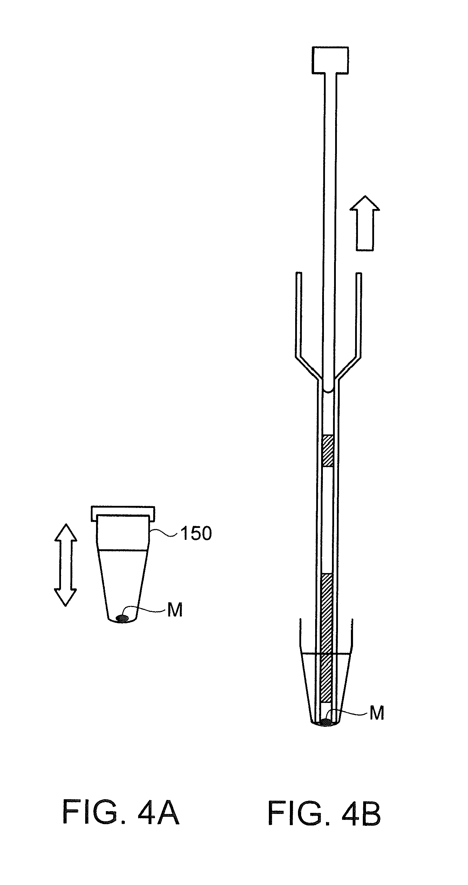

First, as illustrated in FIGS. 4A and 4B, 375 µL of an adsorbent, and 1 µL of a magnetic particle (magnetic particle M) dispersed liquid were contained in a 3-mL polyethylene vessel (adsorption vessel 150). The composition of the adsorbent was 76 mass% guanidine hydrochloride, 1.7 mass% EDTA·2Na dihydrate, and a 10 mass% polyoxyethylene sorbitan monolaurate aqueous solution (MagExtractor -Genome-, NPK-1; Toyobo). The magnetic particle dispersed liquid contained 50 volume% magnetic silica particles, and 20 mass% lithium chloride.

Fifty microliters of blood collected from human was pipetted into the adsorption vessel 150 through the opening. After capping the opening, the adsorption vessel 150 was agitated by shaking it with hand for 30 seconds to adsorb the blood nucleic acid to the magnetic particles M (see FIG. 4A). After removing the cap from the adsorption vessel 150, the opening 140 on the first plug side of the reaction vessel 100 is inserted into the adsorption vessel 150, and the plunger 130 was slid up against the opening 120. This charges the magnetic particles M inside the adsorption vessel 150 into the tube portion 110 of the reaction vessel 100 with the adsorbent (see FIG. 4B). The capillary 12 with the plunger 130 is then installed on the mount section 310 provided in the substance extraction unit 300. Here, the capillary 12 is installed so that the opening 120 side of the tube is vertically above the opening 140. With the reaction vessel 100 installed in place, the opening 140 is past below the permanent magnets 320A and 320B, and the permanent magnets 320A and 320B are on the sides of the capillary 12 at the first plug position. As a result, the magnetic force from the permanent magnets 320A and 320B moves the magnetic particles M to the first plug position inside the capillary 12 (see FIG. 5A). The permanent magnet 320A and the permanent magnet 320B are on the opposite sides of the capillary 12, facing each other with a certain distance in between. The permanent magnets 320A and 320B move reciprocally along the axis orthogonal to the longitudinal direction of the tube so as to vary their distances from the capillary 12.

The liquids inside the tube are moved by pushing the plunger 130 toward the opening 140 while moving the permanent magnets 320A and 320B reciprocally along the axis orthogonal to the tube longitudinal direction. Under the applied pressure of the plunger 130 inside the tube, the second plug 210 of a washing liquid moves to the level that lies on the axis along which the permanent magnets 320A and 320B are undergoing reciprocal movement. This moves the magnetic particles M to the second plug relative to the first plug of oil. The magnetic particles M are washed by the washing liquid, and the foreign substances around the magnetic particles M are removed as the permanent magnets 320A and 320B make reciprocal movement while the plunger 130 sends the liquid (see FIGS. 5B and 5C). As the plunger 130 sends the liquids further, the second plug moves toward the opening 140, and the third plug 220 of the oil comes to lie on the axis along which the permanent magnets 320A and 320B are undergoing reciprocal movement. It is desirable to stop the reciprocal movement of the permanent magnets 320A and 320B while the magnetic particles are passing through the interface between the second plug 210 and the third plug 220 so that the magnetic particles can make passage through the interface while the permanent magnet 320A or 320B is held still proximate to the tube side surface. In this way, it is possible to avoid the magnetic particles M from entering the third plug while the washing liquid forming the second plug is still present in abundance around the magnetic particles M (see FIGS. 5B and 5D). As the plunger 130 sends the liquids further, the third plug moves to the opening 140, and the fourth plug 230 forming the elution liquid comes to lie on the axis along which the permanent magnets 320A and 320B are undergoing reciprocal movement. The sending of the liquids is suspended upon the magnetic particles M having moved to the position near the middle of the fourth plug relative to the tube longitudinal direction, and the permanent magnets 320A and 320B are moved reciprocally while heating the elution liquid with the heating section 340 from the tube side surface to elute the adsorbed nucleic acid from the magnetic particles M into the elution liquid 230 (see FIG. 5E). This completes the elution of the nucleic acid into the elution liquid for amplification reaction. The plunger 130 is then pushed toward the opening 140 to discharge the elution liquid 230 with the eluted nucleic acid through the opening 140 (see FIG. 5F), and this liquid is charged into an amplification reaction vessel with a part of or all of the oil forming the third plug 220 and the fifth plug 240. In this way, the introduction of the liquid with the eluted extract into the amplification reaction vessel can be automated.

### Example of Integrated Nucleic Acid Amplification Device

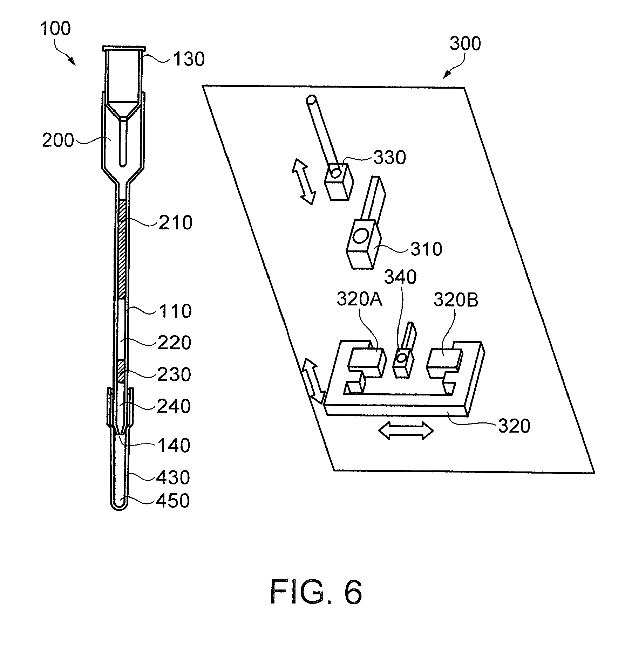

As illustrated in FIG. 6, the nucleic acid purification device of this Example includes a reaction vessel 100 and a substance extraction unit 300. The reaction vessel 100 includes the capillary 12 having a longitudinal direction, and a plunger 130 joined to the opening 120 at the upper end of the capillary 12. The capillary 12 has an opening 140 at the lower end opposite the opening 120, and the reaction vessel 100 has a nucleic acid amplification vessel 430 in communication with the opening 140. The capillary 12 is charged with a first plug 200 of oil, a second plug 210 of a washing liquid, a third plug 220 of oil, a fourth plug 230 of an elution liquid, and a fifth plug 240 of oil, in this order from the opening 120 to the opening 140. The washing liquid and the elution liquid are aqueous solutions immiscible with the oil. The substance extraction unit 300 includes a mount section 310 on which the reaction vessel 100 is mounted, a magnetic force applying mechanism 320 that applies a magnetic force from outside of the reaction vessel 100, permanent magnets 320A and 320B that apply a magnetic force from the side surface of the capillary 12 of the reaction vessel 100, a liquid sending mechanism 330 that pushes the plunger 130 to send the liquid inside the reaction vessel 100, and a heating section 340 that partially heats the capillary 12.

The oil is not particularly limited, and may be, for example, mineral oil, silicone oil (e.g. , 2 CS silicone oil), or vegetable oil. By using a high viscosity oil, it is possible to improve the "wiping effect" of the oil for the nucleic acid-binding solid phase support moving across the interface with the upper plug. In this way, the water-soluble components adhering to the nucleic acid-binding solid phase support do not easily enter the oil when the nucleic acid-binding solid phase support moves into the oil plug from the upper plug.

The washing liquid charged in the second plug is preferably water or a low-salt aqueous solution, and is preferably a buffer in the case of a low-salt aqueous solution. The salt concentration of the low-salt aqueous solution is preferably 100 mM or less, more preferably 50 mM or less, most preferably 10 mM or less. The lower limit of the low-salt aqueous solution concentration is not particularly limited, and is preferably 0.1 mM or more, further preferably 0.5 mM or more, most preferably 1 mM or more. The solution may contain a surfactant such as Triton, Tween, and SDS, and the pH is not particularly limited. The salt used to prepare the buffer is not particularly limited, and salts such as tris, HEPES, PIPES, and phosphates are preferably used. Particularly preferred for use are 8 M guanidine hydrochloride, and 0.7% Triton X-100. The washing liquid preferably contains alcohol in amounts that do no inhibit the adsorption of the nucleic acids to the support, the reverse transcription reaction, and the PCR reaction. The alcohol content is not particularly limited, and may be 70% or less, 60% or less, 50% or less, 40% or less, 30% or less, 20% or less, 10% or less, and is preferably 5% or less, or 2% or less, further preferably 1% or less, or 0.5% or less, most preferably 0.2% or less, or 0.1% or less. The washing liquid may contain a chaotropic agent. For example, containing guanidine hydrochloride in the washing liquid can wash the particles or the like while maintaining or enhancing the adsorption of the nucleic acids to the particles or the like. When containing guanidine hydrochloride, the guanidine hydrochloride content may be, for example, 3 mol/L to 10 mol/L, preferably 5 mol/L to 8 mol/L. With these guanidine hydrochloride content ranges, it is possible to wash other foreign substances or the like while more stably adsorbing nucleic acids to the particles or the like.

The elution liquid of the fourth plug may contain a reverse transcription reagent with reverse transcriptase. The reverse transcriptase is not particularly limited. For example, reverse transcriptases of avian myeloblast virus, ras associated virus type 2, mouse molony murine leukemia virus, and human immunodeficiency virus type 1 origin may be used. The enzyme is preferably heat resistant. The dNTP or salt concentration may be appropriately adjusted to suit for the reverse transcriptase, and is typically 10 to 1000 µM, preferably 100 to 500 µM for dNTP, 1 to 100 mM, preferably 5 to 10 mM for Mg²⁺, and 1 to 2000 mM, preferably 200 to 700 mM for Cl⁻. The total ion concentration is not particularly limited, and may be higher than 50 mM, and is preferably higher than 100 mM, more preferably higher than 120 mM, further preferably higher than 150 mM, even more preferably higher than 200 mM. The upper limit is preferably 500 mM or less, more preferably 300 mM or less, further preferably 200 mM or less. The oligonucleotides for reverse transcription primers are used in 0.1 to 10 µM, preferably 0.1 to 1 µM.

The nucleic acid amplification vessel 430 is filled with oil, and a freeze-dried nucleic acid amplification reaction reagent is held in the oil. Preferably, the freeze-dried nucleic acid amplification reaction reagent is undissolvable in the oil.

The freeze-dried nucleic acid amplification reaction reagent contains at least a DNA polymerase and dNTP, and preferably contains nucleic acid amplification reaction primers and/or nucleic acid amplification reaction probes. When the reverse transcription reagent is not added to the elution liquid of the fourth plug, the freeze-dried nucleic acid amplification reaction reagent may contain a reverse transcription reagent with reverse transcriptase. In this case, the reverse transcription primers may be contained separately from the nucleic acid amplification reaction primers, or may be the same primers used as the nucleic acid amplification reaction primers.

The DNA polymerase is not particularly limited, and is preferably a heat resistant enzyme or a PCR enzyme. For example, Taq polymerase, Tfi polymerase, and Tth polymerase, including the modified forms thereof, are some of the examples of the numerous commercially available products of such enzymes. Preferred is the DNA polymerase for its ability to allow a hot start. The dNTP or salt concentration may be appropriately adjusted to suit for the type of the enzyme used, and is typically 10 to 1000 µM, preferably 100 to 500 µM for dNTP, 1 to 100 mM, preferably 5 to 10 mM for Mg²⁺, and 1 to 2000 mM, preferably 200 to 700 mM for Cl⁻. The total ion concentration is not particularly limited, and may be higher than 50 mM, and is preferably higher than 100 mM, more preferably higher than 120 mM, further preferably higher than 150 mM, even more preferably higher than 200 mM. The upper limit is preferably 500 mM or less, more preferably 300 mM or less, further preferably 200 mM or less. The primer oligonucleotides are used in 0.1 to 10 µM, preferably 0.1 to 1 µM each.

The nucleic acid amplification reaction reagent may be freeze dried by using a common method. For example, the nucleic acid amplification reaction reagent for a single run of reaction is added to a nucleic acid amplification reaction buffer in a container used as the nucleic acid amplification reaction vessel 430, rapidly frozen, and allowed a predetermined time to stand under reduced pressure. The amount of the nucleic acid amplification reaction buffer is not particularly limited, and may be 5 µL, preferably 2.5 µL, more preferably 1.6 µL. The nucleic acid amplification reaction reagent becomes smaller and harder as it adheres to the bottom of the container when the buffer is used in smaller amounts. The rapid freezing temperature is not particularly limited, and is preferably -10°C to -160°C, most preferably about-80°C. For improved solubility, the freeze dried nucleic acid amplification reaction reagent preferably has a form of a sponge or a cake with large numbers of micropores (pores) containing bubbles. The rapid freezing reduces the size of the micropores, and further improves preservability. The average pore size (for example, the average of cross sectional pore diameter measurements performed for certain numbers of times under a microscope) is preferably 20 µm or less. The reduced pressure is not particularly limited, and is preferably 100 mmHg or less, most preferably 20 mmHg or less. The time under reduced pressure is not particularly limited, and is preferably 2 to 24 hours, most preferably about 8 hours. The amount of the nucleic acid amplification reaction reagent solution used to freeze dry the nucleic acid amplification reaction reagent should preferably be smaller than the amount of the aqueous solution used to dissolve the nucleic acid amplification reaction reagent.

The nucleic acid amplification reaction reagent is freeze dried in the container used as the nucleic acid amplification reaction vessel 430, and allowed to adhere to the bottom of the container. This is followed by adding oil, preferably by filling the container. Preferably, the oil is dehydrated with a silica gel or the like in advance, and added in a glove box or the like because the freeze dried nucleic acid amplification reaction reagent is susceptible to water, and cannot be stably preserved for extended time periods in the presence of water. The container should preferably be gently centrifuged to remove bubbles after adding oil.

The freeze dried nucleic acid amplification reaction reagent can be prevented from diffusing in the oil by adding and solidifying a dissolved wax before adding the oil to the freeze dried nucleic acid amplification reaction reagent. Here, "wax" means an organic material that is solid at room temperature, and that turns to liquid upon being heated. Preferably, the wax used in the invention has a melting point of 31°C or more, preferably 36°C or more, more preferably 41°C or more, further preferably 46°C or more, and 100°C or less, preferably 90°C or less, more preferably 80°C or less, further preferably 70°C or less, and is made of materials such as neutral fats, higher fatty acids, and hydrocarbons. The wax is not particularly limited. Examples include petroleum-derived waxes such as paraffin, and microcrystalline waxes; animal-derived waxes such as beeswax, wool wax, and spermaceti; and plant-derived waxes such as carnauba, rosin, Candelilla wax, and sumac wax. Other examples include El Crysta^{®} (Idemitsu Kosan), Nissan Electol^{®} (NOF Corporation), Poem^{®} (Riken Vitamin), Rikemal^{®} (Riken Vitamin), Neowax^{®} (Yasuhara Chemical), Hi-Wax^{®} (Mitsui Chemicals), and Silicone Wax^{®} (Dow Corning Toray Co., Ltd.).

The following specifically describes a method for amplification of nucleic acids from a specimen collected with a cotton swab using the nucleic acid purification device.

First, the reaction vessel 100 is installed in the substance extraction unit 300 so that the opening 120 side of the tube is vertically above the opening 140. With the reaction vessel 100 installed in place, the permanent magnets 320A and 320B are on the sides of the plunger 130. The permanent magnet 320A and the permanent magnet 320B are on the opposite sides of the capillary 12, facing each other with a certain distance in between. The permanent magnets 320A and 320B can move reciprocally along the axis orthogonal to the longitudinal direction of the tube so as to vary their distances from the capillary 12. In the initial setting, the permanent magnet 320A is closer to the capillary 12.

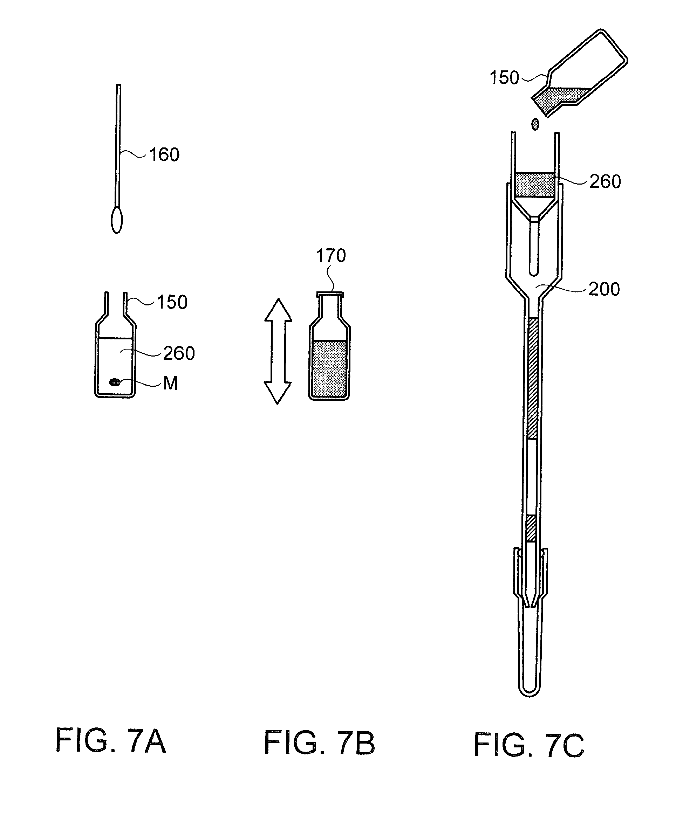

Thereafter, as shown in FIG. 7A, 375 µL of adsorbent 260 is charged into a 3-mL polyethylene container (adsorption vessel 150), and 1 µL of a magnetic particle (magnetic particle M) dispersion is added. The adsorbent 260 is an aqueous solution of the composition containing 76 mass% of guanidine hydrochloride, 1.7 mass% of EDTA·2Na dihydrate, and 10 mass% of a polyoxyethylene sorbitan monolaurate (MagExtractor -Genome-, NPK-1; Toyobo). The magnetic particle dispersion contains 50 volume% of magnetic silica particles, and 20 mass% of lithium chloride.

A cotton swab 160 with the specimen from human is immersed in the adsorbent 260. After placing a cap 170, the adsorption vessel 150 is agitated by shaking it with hand for 30 seconds to adsorb the nucleic acids in the specimen to the magnetic particles M (see FIG. 7B. After removing the cap 170 from the adsorption vessel 150, the reaction vessel 100 is inserted into the adsorption vessel 150 at the opening 120 on the first plug side, and the content is added into the plunger 130 of the reaction vessel 100 installed in the substance extraction unit 300 (see FIG. 7C).

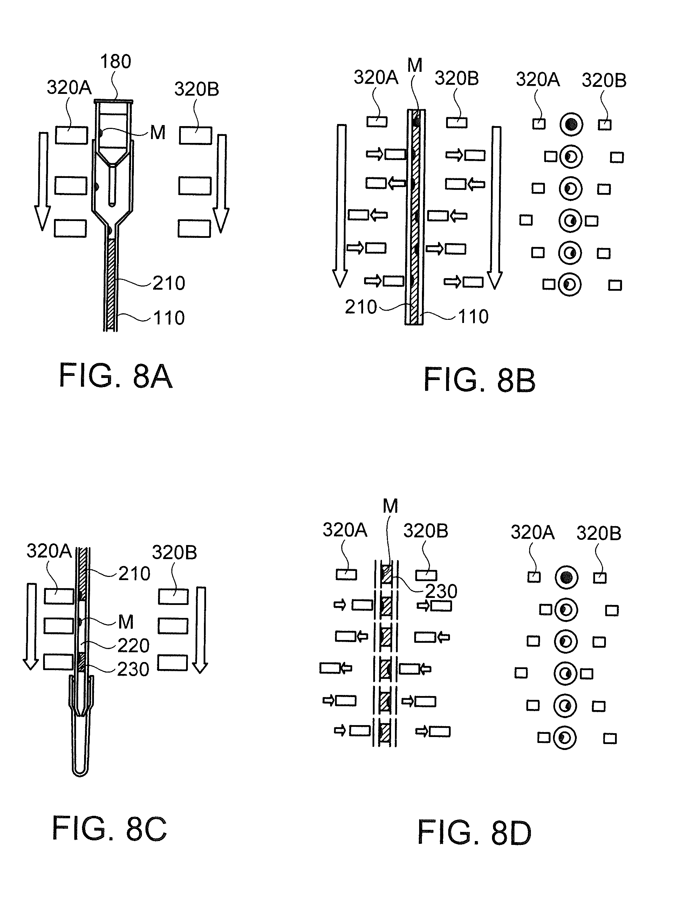

As shown in FIG. 8A, this charges the magnetic particles M inside the adsorption vessel 150 into the plunger 130 with the adsorbent, and the magnetic particles M adhere to the side surface of the plunger 130 under the magnetic force from the permanent magnet 320A. Thereafter, a cap 180 is placed on the plunger 130, and the permanent magnet 320A is vertically moved straight down to vertically move the magnetic particles M down into the capillary 12. The magnetic particles M move past the first plug 200 of oil, and enter the second plug 210 of a washing liquid. The permanent magnets 320A and 320B are then vertically moved further down while being swung. Specifically, the magnetic particles M are attracted by whichever of the permanent magnets 320A and 320B is closer to the magnetic particles M, provided that the permanent magnets 320A and 320B have substantially the same magnetic force. The magnetic particles M are thus attracted to the permanent magnet 320A when it is closer to the capillary 12. As the permanent magnet 320A is moved away from the tube 20, the magnetic particles M are attracted by the permanent magnet 320B approaching the capillary 12 from the opposite side. In this way, it is possible to swing the magnetic particles M inside the capillary 12 by alternately swinging the permanent magnets 320A and 320B sideways relative to the side surface of the capillary 12. The magnetic particles M can thus be moved vertically down while undergoing swinging movements (see FIG. 8B). Preferably, this reciprocating motion is performed at least twice for each plug. When the permanent magnets 320A and 320B have different magnetic forces, the magnetic particles M move toward the permanent magnet of the greater magnetic force. The magnetic particles M also can be swung in the same fashion by taking into consideration such a magnetic force difference.

Upon the magnetic particles M entering the third plug 220 of oil, the swing motion of the permanent magnets 320A and 320B is stopped, and the permanent magnets 320A and 320B are vertically moved straight down. This movement moves the magnetic particles M vertically down along the wall of the capillary 12 (see FIG. 8C).

Upon the magnetic particles M entering the fourth plug 230 of an elution liquid, the permanent magnets 320A and 320B are moved vertically down while being swung (see FIG. 8D) . The nucleic acids bound to the magnetic particles M elute in this process. Here, it is preferable for improved elution efficiency to reciprocally move the magnetic particles M in the longitudinal direction of the fourth plug 230. The elution of nucleic acids also can be accelerated by heating with the heating section 340. When the extracted nucleic acid is RNA, and the elution liquid of the fourth plug 230 contains a reverse transcription reagent, a reverse transcription reaction is performed at this stage by heating the fourth plug 230 with the heating section 340. The reaction conditions, including reaction temperature and reaction time, may be selected to suit for the reverse transcriptase.

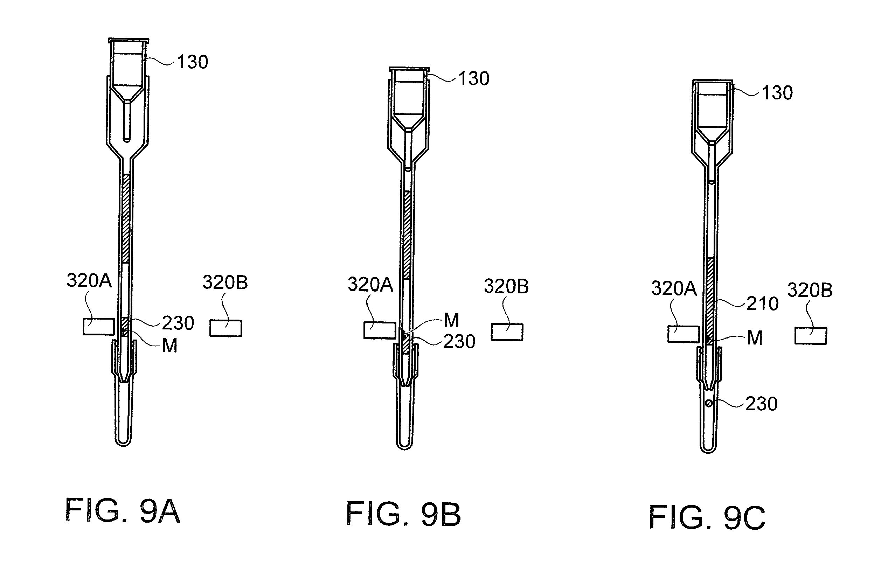

Thereafter, the permanent magnet 320A is stopped while it is close to the tube, as shown in FIG. 9A, and the liquid inside the tube is moved by pushing the plunger 130 toward the opening 140 side in the manner shown in FIG. 9B. Under the applied pressure of the plunger 130 inside the tube, each plug moves downward while the permanent magnet 320A remains still. As the plunger 130 continuously sends the liquid, the fourth plug 230 moves toward the opening 140 side into the nucleic acid amplification vessel 430, as shown in FIG. 9C. The elution liquid of the fourth plug 230 forms droplets in the oil inside the nucleic acid amplification vessel 430. The droplets of elution liquid dissolve the freeze dried nucleic acid amplification reaction reagent inside the nucleic acid amplification vessel 430, and produce a nucleic acid amplification reaction liquid.

When the extracted nucleic acid is RNA, and the elution liquid of the fourth plug 230 contains a reverse transcription reagent, the elution liquid contains the cDNA produced by reverse transcription. The elution liquid contains RNA when the reverse transcription reagent is not contained. In this case, cDNA can be synthesized by reverse transcription of the RNA in the nucleic acid amplification vessel 430 when the freeze dried nucleic acid amplification reaction reagent contains a reverse transcription reagent. The elution liquid contains DNA when the extracted nucleic acid is DNA. A nucleic acid amplification reaction may be performed with the freeze dried nucleic acid amplification reaction reagent by using the DNA as a template.

When the nucleic acid amplification reaction vessel 430 containing the nucleic acid amplification reaction reagent and the oil can be used by being directly set in a nucleic acid amplification device such as a PCR apparatus, the nucleic acid amplification reaction vessel 430 directly can be used for nucleic acid amplification reaction by removing it after the nucleic acid amplification reaction reagent has dissolved in the elution liquid. Alternatively, when the nucleic acid amplification device accommodates direct use of the reaction vessel 100, the reaction vessel 100 directly can be used for nucleic acid amplification reaction after the nucleic acid amplification reaction reagent has dissolved in the elution liquid.

## Claims

1. A target substance purification device comprising:
a mount section adapted to mount a capillary that has a longitudinal direction and includes a plug of oil, a plug of an elution liquid that undergoes phase separation from oil, and is configured to elute the target substance from a magnetic body detachably retaining the target substance, and a plug of oil in this order inside the capillary;
a magnetic force applying mechanism that is configured to apply a magnetic force to the capillary to retain the magnetic body, and control the movement of the magnetic body along the longitudinal direction of the capillary; and
a liquid sending mechanism that is configured to move the elution liquid along the longitudinal direction of the capillary.

2. A target substance purification device comprising:
a capillary that has a longitudinal direction and includes a plug of oil, a plug of an elution liquid that undergoes phase separation from oil, and is configured to elute the target substance from a magnetic body detachably retaining the target substance, and a plug of oil in this order inside the capillary;
a magnetic force applying mechanism that is configured to apply a magnetic force to the capillary to retain the magnetic body, and control the movement of the magnetic body along the longitudinal direction of the capillary; and
a liquid sending mechanism that is configured to move the elution liquid in the longitudinal direction of the capillary while the magnetic force applying mechanism is restricting the movement of the magnetic body along the longitudinal direction of the capillary.

3. The target substance purification device according to claim 1 or 2, wherein the magnetic force applying mechanism includes a permanent magnet.

4. The target substance purification device according to any one of claims 1 to 3 , wherein the liquid sending mechanism includes a pressure applying member.

5. The target substance purification device according to claim 4, wherein the pressure applying member is a plunger.

6. A nucleic acid purification device comprising:
a mount section adapted to mount a capillary that has a longitudinal direction and includes an oil plug of oil, a washing liquid plug of a washing liquid, an oil plug of oil, an elution liquid plug of an elution liquid that undergoes phase separation from oil, and is configured to elute a nucleic acid from a magnetic body detachably retaining the nucleic acid, and an oil plug of oil in this order inside the capillary;
a magnetic force applying mechanism that is configured to apply a magnetic force to the capillary to retain the magnetic body, and control the movement of the magnetic body along the longitudinal direction of the capillary; and
a liquid sending mechanism that is configured to move the elution liquid plug along the longitudinal direction of the capillary.

7. The nucleic acid purification device according to claim 6, wherein the magnetic force applying mechanism is configured to move the magnetic body within a plane that crosses the longitudinal direction of the capillary.

8. The nucleic acid purification device according to claim 6 or 7, comprising a nucleic acid amplification vessel in communication with inside of the capillary.

9. The nucleic acid purification device according to claim 8, wherein the nucleic acid amplification vessel contains a freeze dried nucleic acid amplification reagent.

10. A method for purifying a target substance,
the method comprising:
introducing a magnetic body retaining the target substance into a capillary that has a longitudinal direction and includes a plug of oil, an elution liquid plug of an elution liquid that undergoes phase separation from oil, and is configured to elute the target substance from a magnetic body detachably retaining the target substance, and a plug of oil in this order inside the capillary;
externally applying a magnetic force to the capillary to move the magnetic body into the elution liquid plug and retain the magnetic body inside the elution liquid plug;
eluting the target substance from the magnetic body in the elution liquid; and
moving the elution liquid with the eluted target substance out of the capillary in the longitudinal direction of the capillary while restricting the movement of the magnetic body along the longitudinal direction of the capillary.

11. The method according to claim 10, comprising moving the magnetic body within a plane that crosses the longitudinal direction of the capillary.

12. The method according to claim 10 or 11, wherein the elution liquid with the eluted target substance is moved in the longitudinal direction of the capillary by increasing the pressure inside the capillary.

13. The method according to claim 12, wherein the pressure inside the capillary is increased with a plunger.

14. A method for amplifying a nucleic acid obtained as the target substance by using the method of any one of claims 10 to 13.
